# EUROPÄISCHE PATENTSCHRIFT

(11) **EP 0 997 735 B1**
(45) Veröffentlichungstag und Bekanntmachung des Hinweises auf die Patenterteilung: **30.01.2008**
(21) Anmeldenummer: 99119336.8
(22) Anmeldetag: 29.09.1999
(51) Int. Cl.: G01N 33/96

(54) **Stabilisierung von biologischen Flüssigkeiten durch Zusatz von Sterinestern**
Stabilisation of biological fluids by addition of sterol esters
Stabilisation des fluides biologiques par l'ajout d'esters stéroliques

(30) Priorität: 30.10.1998 DE 19850074
(43) Veröffentlichungstag der Anmeldung: 03.05.2000
(73) Patentinhaber: Dade Behring Marburg GmbH, 35001 Marburg (DE)
(72) Erfinder: Althaus, Harald, 35083 Wetter (DE)

(56) Entgegenhaltungen:
- WO-A-00/51572
- WO-A-97/37637
- US-A- 4 127 502
- PROKSCH G J ET AL: "A WATER SOLUBLE CHOLESTEROL DERIVATIVE FOR USE IN AUGMENTING SERUM CONTROL MATERIALS" CLINICAL CHEMISTRY, Bd. 24, Nr. 11, 1978, Seiten 1924-1926, XP009004439 ISSN: 0009-9147
- LAUTERIO T J ET AL: "GROWTH, PROTEIN SYNTHESIS AND PLASMA CONCENTRATIONS OF GROWTH HORMONE, THYROXINE AND TRIIODOTHYRONINE IN DWARF, CONTROL AND GROWTH-SELECTED STRAINS OF BROILER-TYPE DOMESTIC FOWL" COMPARATIVE BIOCHEMISTRY AND PHYSIOLOGY. PART A. COMPARATIVE PHYSIOLOGY, ELSEVIER SCIENCE LTD, XX, Bd. 83A, Nr. 4, 1986, Seiten 627-632, XP000671975 ISSN: 0300-9629
- PROKSCH G.J.; BONDERMAN D.P.: 'PREPARATION OF OPTICALLY CLEAR LYOPHILIZED HUMAN SERUM FOR USE IN PREPARING CONTROL MATERIAL' CLINICAL CHEMISTRY Bd. 22, Nr. 4, 1976, WASHINGTON, DC, US, Seiten 456 - 460

## Beschreibung

Die vorliegende Erfindung betrifft die Verwendung von Sterinestern zur Langzeitstabilisierung von biologischen Flüssigkeiten, insbesondere auch solchen, die durch Lyophilisation und anschließende Rekonstitution erhalten werden.

Bei allen Arbeiten mit biologischen Flüssigkeiten, insbesondere bei solchen, an denen oder mit Hilfe derer optisch auszuwertende Untersuchungen vorgenommen werden sollen, stellt sich das Problem der Trübung. Die Ursache dieser Trübungen ist nicht immer nachweisbar, oft sind es Protein- oder Lipidabscheidungen aus der Lösung. Mitverantwortlich für diese Instabilitäten werden die in Körperflüssigkeiten vorkommenden Lipoprotein-Makromoleküle gemacht, die aufgrund ihres Gehaltes an wasserunlöslichen Phospholipiden per se eine Tendenz zur Aggregation aufweisen.

Werden labile Proteinlösungen gelagert, so kann es ebenfalls zu Eintrübungen oder Adsorption der Proteine an Gefäßwandungen kommen, welche die Qualität dieser Produkte erheblich verschlechtern.

Die oben genannten Instabilitäten verstärken sich noch, wenn die biologischen Flüssigkeiten zur Stabilisierung lyophilisert werden. Beispiele von biologischen Flüssigkeiten sind Serum, Plasma, Liqour, Pleuralexudate oder Ascites menschlichen oder tierischen Ursprungs.

Im Sinne der Erfindung können solche biologischen Flüssigkeiten auch synthetisch zusammengesetzte Lösungen aus einer dem Fachmann in sich bekannten artifiziellen oder auch natürlichen Flüssigkeitsmatrix (z. B. Serum oder Phosphatgepufferter NaCl-Lösung) und zugesetzten biologischen Substanzen, die ihrerseits gentechnisch hergestellt sein können, sein.

Solche synthetisch zusammengesetzten Lösungen werden häufig als Kontroll- oder Standardseren verwendet.

Unter Kontrollseren sind Seren humanen oder tierischen Ursprungs mit gegebenenfalls veränderter, jedoch serumähnlicher Zusammensetzung zu verstehen, die Serumbestandteile, beispielsweise Proteine, Enzyme, enzymatisch bestimmbare Substrate und Elektrolyte in bekannter Konzentration enthalten und zur Kontrolle von Bestimmungsmethoden für diese Serumbestandteile geeignet sind.

Um die Haltbarkeit labiler Komponenten wie beispielsweise Enzymen oder Lipoproteinen zu gewährleisten, können biologische Flüssigkeiten lyophilisiert und/oder bei niedriger Temperatur, bevorzugterweise unter -18 °C, gelagert werden. Unerwünschte Nebeneffekte einer Lyophilisation sind Trübungen, die nach Rekonstitution der Kontrollseren durch Veränderung des Lösungsverhaltens vor allem der Lipoproteine auftreten. Diese Trübungen stören häufig bei spektrophotometrischen Methoden, so daß z. B. ein Probenleerwert zusätzlich erforderlich ist.

Da Probleme durch Trübung häufig auftreten, wurden auch schon zahlreiche Versuche zur Beseitigung unternommen. Auch wenn schon Lösungen gefunden wurden, waren diese jedoch bisher im wesentlichen auf eng umgrenzte Anwendungsgebiete und Bedingungen beschränkt.

In DE-P 31 07 060 ist der Zusatz organischer nicht zuckerartiger Substanzen wie Methanol, Alanin, Triäthylenglycol, Valin, Acetat, Lactat oder Natrium-2-hydroxymethylbutyrat beschrieben. Ein solcher Zusatz kann z. B. im Falle von Alanin und Methylbutyrat Störungen von Enzymreaktionen zur Folge haben.

Ein Methanolzusatz ist allgemein gesundheitsgefährdend. Wenn Natriumacetat verwendet wird, ist das Kontrollserum nicht mehr als Universal-Kontrollserum für Elektrolytbestimmungen einsetzbar. Ein Zusatz von Ammoniumverbindungen stört bei Harnstoff-Bestimmungen . Andere Substanzen können generelle Teststörungen verursachen.

Ein weiteres Verfahren zur Vermeidung von Trübungen ist in DE-OS 33 29 952 durch Zusatz von Prolin und Na-Desoxycholat vorbeschrieben. Dieser Zusatz hat jedoch den Nachteil, daß er z. B. zu Artefakten bei der Proteinauftrennung führt und die Enteiweißung mit Trichloressigsäure z. B. bei Bestimmung von Kreatinin erschwert.

Der vorliegenden Erfindung lag daher die Aufgabe zugrunde, ein Verfahren zu finden, das generell zur Langzeitstabilisierung von biologischen Flüssigkeiten angewendet werden kann.

Überraschenderweise konnte durch Zusatz von Sterinestern zu biologischen Körperflüssigkeiten eine Stabilisierung auch in Gegenwart von Lipoproteinen erzielt werden. Auch nach Lyophilisation mit Sterinestern versetzter lipoproteinhaltiger Körperflüssigkeiten, erhält man nach Auflösen ein klares Produkt.

Überraschenderweise konnte auch festgestellt werden, daß labile Proteinlösungen, die zu Eintrübungen neigen oder deren Proteine leicht an Gefäßwandungen adsorbieren, durch Zusatz von Sterinestern stabilisiert werden.

Überraschenderweise wurde ferner gefunden, daß sich insbesondere auch trübungsärmere, homogenere rekonstituierte Kontrollsera herstellen lassen, wenn dem Kontrollserum vor der Lyophilisation Sterinester zugesetzt wird. Dadurch wird das Serum insgesamt nicht nur homogener sondern auch die Präzision von Konzentrations- und Aktivitätsbestimmungen der im Kontrollserum enthaltenen Parameter verbessert, d. h. die Wiederfindung der deklarierten Sollwerte wird für den Anwender von Qualitätskontrollsera erleichtert.

Sterinester gehören zu der Stoffklasse der Steroide (Gonanderivate) die allgemein eine Hydroxygruppe in der 3 ß Position kennzeichnet. Wesentliche Unterschiede bestehen in der in 17(20) Stellung haftenden Seitenkette. Sterine stellen eine große Klasse dar (BEYER et al. (1981), Lehrbuch der organischen Chemie, S. 649-664 "Steroide"). Die mit unterschiedlichen Vertretern dieser Klasse - z. B. Vitamin D3, Oestron, Cholesterin und Stigmasterin - durchgeführten Experimente zeigen, daß eine generelle Anwendbarkeit gewährleistet ist. Besonders vorteilhaft sind die Derivate des Cholesterins.

Die erfindungsgemäßen Sterinester besitzen außerdem eine über eine Dicarbonsäure gekoppelte Polyethylenglykolgruppe. Prinzipiell können alle bekannten Dicarbonsäuren verwendet werden, da für die erfindungsgemäße Funktion im wesentlichen die bifunktionale Reaktivität ausschlaggebend ist . Vorteilhafterweise können folgende Dicarbonsäuren verwendet werden: Bernsteinsäure, Adipinsäure, Sebacinsäure.

Die PEG Gruppe soll im wesentlichen auch die Löslichkeit des Sterinesters gewährleisten, so daß der Fachmann, gegebenenfalls durch ein Experiment, die optimale Länge leicht bestimmen kann. Erfahrungsgemäß sind folgende Kettenlängen vorteilhaft: Polyethylenglycol 600, Polyethylenglycol 900 oder Polyethylenglycol 3000.

Als Maß für den Stabilisierungseffekt dient vorteilhafterweise das Trübungsverhalten gemessen in einem nephelometrischen Meßverfahren. Es ist für den Fachmann anhand der vorliegenden Erfindung leicht, ein anderes, entsprechendes Verfahren einzusetzen.

Gegenstand der Erfindung ist somit ein Verfahren zur Stabilisierung von biologischen Flüssigkeiten sowie die so stabilisierten Flüssigkeiten.

Bevorzugt zur Langzeitstabilisierung von biologischen Flüssigkeiten ist dabei die Verwendung von Sterinester der allgemeinen Formel I

R₁-O -(O)C-R₂-C(O)-O-[CH₂-CH₂-O]ₙ-CH₂-CH₂-OH Formel I

worin
n = 1-200 und
R₁ = Sterin ist
R₂ = aliphatischer oder aromatischer Ring mit 4 bis 8 C-Atomen, von denen mindestens eines durch N, S. oder O ersetzt werden kann, oder eine geradlinige oder verzweigte Kette mit 0 bis 12 C-Atomen dar stellt, besonders bevorzugt ist eine Verwendung der Sterinester in denen R₁ eine Verbindung der allgemeinen Formel II ist: 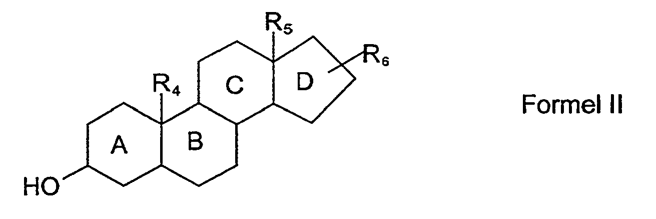
R₄ und R₅ H oder -CH₃ sein können
R₆ eine geradkettige oder verzweigte Kette mit 1 bis 12 C-Atomen, eine -OH oder =O Gruppe sein kann,
die Ringe A,B, C und D jeder für sich gesättigt, ungesättigt, oder aromatisch sein können
und, wenn R₄ = -C(19)H₃ ist, der Ring B zwischen C(9) und C(10) unter Ausbildung einer Doppelbindung zwischen C(9) und C(19) geöffnet sein kann.

Ganz besonders bevorzugt ist die Verwendung von Sterinestern, wobei der Sterinrest von Cholesterin, Vitamin D3, Stigmasterin oder Oestron stammt.

Vorteilhafterweise wird der Sterinester in einer solchen Konzentration zugesetzt wird, daß die Konzentration in der biologischen Flüssigkeit 0.05 - 5 Gew. %, bevorzugterweise 0.1-3 Gew. %, besonders bevorzugterweise 0.5 - 1.5 Gew. % beträgt.

Bevorzugterweise wird die biologische Flüssigkeit nach dem Fachmann an sich bekannten Verfahren lyophilisiert und zum Gebrauch rekonstituiert.

Die biologische Flüssigkeit kann auch bis zum Gebrauch eingefroren, vorteilhafterweise bei ≤ -18 °C, aufbewahrt werden.

Gegenstand der Erfindung ist auch ein Reagenz, im wesentlichen bestehend aus einer natürlichen oder artifiziellen Matrix, mindestens einer diagnostisch relevanten Substanz (Analyt) und einem Sterinester der allgemeinen Formel I in einer Konzentration von 0,05 bis 5 Gew. %.

Zur Erzielung des erfindungsgemäßen Effektes können die erfindungsgemäßen Sterinester auch mit den bekannten Stabilisatoren kombiniert werden, insbesondere mit Detergenzien.

Besonders vorteilhaft sind dabei Kombinationen von Sterinester mit nichtionischen und/oder zwitter-ionischen Detergenzien. In solchen Kombinationen kann die Sterinesterkonzentration vorteilhafterweise verringert werden.

Biologische Flüssigkeiten im Sinne dieser Erfindung werden bereits weiter vorne beschrieben.

Der Begriff der Langzeitstabilität ist u.a. auch von dem Meßverfahren und dem Analyten abhängig. Grundsätzlich sollte die Stabilität jedoch mindestens 6 Monate, bevorzugterweise mindestens 12 Monate, besonders bevorzugterweise mindestens 18 Monate betragen. Eine biologische Flüssigkeit wird dann als stabil im Sinne der vorliegenden Erfindung bezeichnete, wenn die Trübung gemessen bei 546 nm gegenüber der Trübung der Ausgangsflüssigkeit um nicht mehr als 300 % zugenommen hat.

Die folgenden Beispiele erläutern die Erfindung ohne sie einzuschränken.

### Beispiel 1

Citratplasma eines gesunden Spenders wurde mit verschiedenen Konzentrationen Cholesterin-PEG900 (FLUKA CH-9471 Buchs/Schweiz, Art.Nr. 26735) (CP) versetzt und anschließend nach dem Fachmann an sich bekannten Verfahren lyophilisiert.
Zur Dokumentation der Trübung wurden nach Rekonstitution des Lyophilisates der Blindwert am Behringnephelometer (BNA, Dade Behring Marburg GmbH, D-35001 Marburg/Deutschland) und die Extinktion bei 546 nm gemessen.

| | Extinktion 546 nm | Blindwert BNA in Bit |
|---|---|---|
| Ausgangsmaterial: Citratplasma | 0,131 | 11 |
| (ohne Zusatz) | 1,151 | > 3500 |
| + 0,2 % CP | 0,643 | 3257 |
| + 0,5 % CP | 0,643 | 683 |
| + 1 % CP | 0,165 | 84 |

### Beispiel 2

Humanserum wurde mit verschiedenen Konzentrationen Cholesterin-PEG900 (CP) versetzt und bei verschiedenen Temperaturen gelagert (2-8 °C, -20 °C) bzw. lyophilisiert.

Zur Dokumentation der Trübung wurden der Blindwert am Behringnephelometer (BNA) und die Extinktion bei 546 nm gemessen.

| | Humanserum unbehandelt | | Humanserum + 1 % CP | |
|---|---|---|---|---|
| | Extinktion 546 nm | Blindwert BNA in Bit | Extinktion 546 nm | Blindwert BNA in Bit |
| Startwerte | 0,131 | 98 | 0,130 | 98 |
| 4 Tage bei 2-8 °C | 0,215 | 952 | 0,152 | 132 |
| 4 Tage -20 °C | 0,235 | 581 | 0,171 | 155 |
| 1 Jahr bei -20 °C | 0,790 | >3500 | 0,279 | 1837 |
| Lyophilisat (1 Jahr bei 2-8 °C gelagert) nach Rekonstitution | 1,630 | >3500 | 0,355 | 1676 |

## Patentansprüche

1. Verwendung von Sterinester der allgemeinen Formel I
R₁-O -(O)C-R₂-C(O)-O-[CH₂-CH₂-O]ₙ-CH₂-CH₂ -OH
worin
n = 1-200 und
R₁ = Sterin ist
R₂ = aliphatischer oder aromatischer Ring mit 4 bis 8 C-Atomen, von denen mindestens eines durch N, S oder O ersetzt werden kann, oder eine geradlinige oder verzweigte Kette mit 0 bis 12 C-Atomen darstellt
zur Stabilisierung von biologischen Flüssigkeiten.

2. Verwendung gemäß Anspruch 1, wobei R₁ eine Verbindung der allgemeinen Formel II ist: 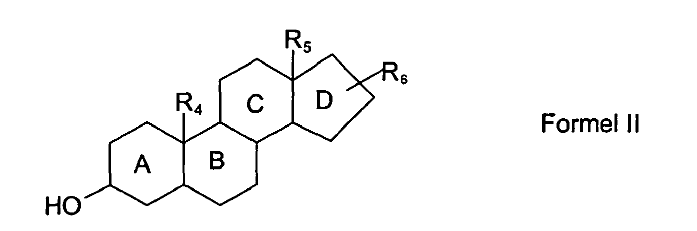
R₄ und R₅ H oder -CH₃ sein können
R₆eine geradkettige oder verzweigte Kette mit 1 bis 12 C-Atomen, eine -OH oder =O Gruppe sein kann.
die Ringe A,B, C und D jeder für sich gesättigt, ungesättigt, oder aromatisch sein können
und, wenn R₄ = -C(19)H₃ ist, der Ring B zwischen C(9) und C(10) unter Ausbildung einer Doppelbindung zwischen C(9) und C(19) geöffnet sein kann.

3. Verwendung gemäß Anspruch 1, wobei R₁ ausgewählt ist aus der Gruppe: Cholesterin, Vitamin D3, Stigmasterin und Oestron.

4. Verwendung gemäß Anspruch 1, **dadurch gekennzeichnet, daß** der Sterinester in einer solchen Konzentration zugesetzt wird, daß die Konzentration in der biologischen Flüssigkeit 0,05 Gew. % bis 5 Gew. % beträgt.

5. Verwendung gemäß einem der Ansprüche 1 bis 4, **dadurch gekennzeichnet, daß** die biologische Flüssigkeit lyophilisiert und zum Gebrauch rekonstituiert wird.

6. Verwendung gemäß einem der Ansprüche 1 bis 4, **dadurch gekennzeichnet, daß** die biologische Flüssigkeit bis zum Gebrauch eingefroren, vorteilhafterweise bei ≤ -18 °C, aufbewahrt wird.

7. Verwendung gemäß einem der Ansprüche 1 bis 6, **dadurch gekennzeichnet, daß** die Stabilisierungsdauer mindestens 6 Monate beträgt.

8. Verwendung gemäß Anspruch 7, **dadurch gekennzeichnet, daß** die Stabilisierungsdauer mindestens 12 Monate beträgt.

9. Verwendung gemäß Anspruch 7, **dadurch gekennzeichnet, daß** die Stabilisierungsdauer mindestens 18 Monate beträgt.

## Claims

1. The use of sterol esters of the general formula I
R₁-O-(O)C-R₂-C(O)-O-[CH₂-CH₂-O]ₙ-CH₂-CH₂-OH
in which
n = 1-200 and
R₁ = sterol
R₂ = aliphatic or aromatic ring having 4 to 8 C atoms, of which at least one can be replaced by N, S or O, or is a linear or branched chain having 0 to 12 C atoms
for the stabilization of biological fluids.

2. The use as claimed in claim 1, where R₁ is a compound of the general formula II: 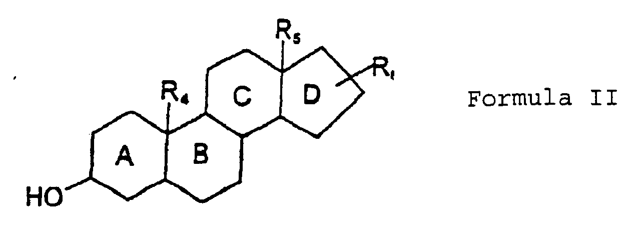
R₄ and R₅ can be H or -CH₃
R₆ can be a straight-chain or branched chain having 1 to 12 C atoms, an -OH or =O group,
the rings A, B, C and D can each be saturated, unsaturated or aromatic per se
and, if R₄ = -C(19)H₃, the ring B between C(9) and C (10) can be opened with formation of a double bond between C(9) and C(19).

3. The use as claimed in claim 1, where R₁ is selected from the group consisting of: cholesterol, vitamin D3 ; stigmasterol and estrone.

4. The use as claimed in claim 1, wherein the sterol ester is added in such a concentration that the concentration in the biological fluid is 0.05% by weight to 5% by weight.

5. The use as claimed in one of claims 1 to 4, wherein the biological fluid is lyophilized and reconstituted for use.

6. The use as claimed in one of claims 1 to 4, wherein the biological fluid is frozen until use, advantageously stored at ≤-18°C.

7. The use as claimed in one of claims 1 to 6, wherein the stabilization period is at least 6 months.

8. The use as claimed in claim 7, wherein the stabilization period is at least 12 months.

9. The use as claimed in claim 7, wherein the stabilization period is at least 18 months.

## Revendications

1. Utilisation d'esters de stérols de formule générale I
R₁O-(O)C-R₂-C(O)-O-[CH₂-CH₂-O]ₙ-CH₂-CH₂-OH
dans laquelle
n = 1-200 et
R₁ = stérol et
R₂ un cycle aliphatique ou aromatique ayant de 4 à 8 atomes de carbone, dont au moins un peut être remplacé par N, S ou O, ou une chaîne linéaire ou ramifiée ayant de 0 à 12 atomes de carbone
pour la stabilisation de liquides biologiques.

2. Utilisation selon la revendication 1, dans laquelle R₁ est un composé de formule générale II: 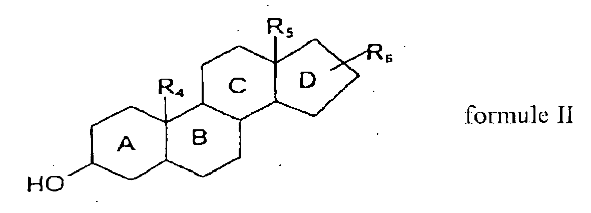
R₄ et R₅ peuvent être H ou -CH₃
R₆ peut être une chaîne linéaire ou ramifiée ayant de 1 à 12 atomes de carbone, un groupe -OH ou =O,
Chacun des cycles A, B, C et D peut être saturé, insaturé ou aromatique
et lorsque R₄ est -C(19)H₃, le cycle B entre C(9) et C(10) peut être ouvert avec formation d'une double liaison entre C(9) et C(19).

3. Utilisation selon la revendication 1 dans laquelle R₁ est choisi dans le groupe suivant : cholestérol, vitamine D3, stigmastérol et oestrone.

4. Utilisation selon la revendication 1, **caractérisée en ce que** l'ester de stérol est ajouté à une concentration telle que la concentration dans le liquide biologique est de 0,05 % en poids à 5 % en poids.

5. Utilisation selon l'une quelconque des revendications 1 à 4, **caractérisée en ce que** le liquide biologique est lyophilisé et reconstitué lors de emploi.

6. Utilisation selon l'une quelconque des revendications 1 à 4, **caractérisée en ce que** le liquide biologique est conservé en l'état congelé, jusqu'a son emploi avantageusement à ≤-18 °C.

7. Utilisation selon l'une quelconque des revendications 1 à 6, **caractérisée en ce que** la durée de la stabilisation est d'au moins 6 mois.

8. Utilisation selon la revendication 7, **caractérisée en ce que** la durée de la stabilisation est d'au moins 12 mois.

9. Utilisation selon la revendication 7, **caractérisée en ce que** la durée de la stabilisation est d'au moins 18 mois.
